# EUROPEAN PATENT SPECIFICATION

(11) **EP 2 134 446 B1**
(45) Date of publication and mention of the grant of the patent: **02.09.2015**
(21) Application number: 08741736.6
(22) Date of filing: 06.03.2008
(51) Int. Cl.: B01D 53/04, B01D 53/26, B01D 53/14, C10L 3/10

(54) **BIOGAS UPGRADING**
BIOGASVEREDELUNG
AMÉLIORATION DE BIOGAZ

(30) Priority: 20.03.2007 NZ 55399207
(43) Date of publication of application: 23.12.2009
(73) Proprietor: PT Biogas Technology Limited, Sheffield S9 1BT (GB)
(72) Inventor: BETHELL, Warwick, 11668 Stockholm (SE)
(74) Representative: Boult Wade Tennant
(86) International application number: PCT/NZ2008/000043
(87) International publication number: WO 2008/115079

(56) References cited:
- EP-A1- 0 180 670
- EP-A1- 1 120 149
- EP-A1- 1 811 011
- EP-B1- 0 612 554
- WO-A2-2004/058630
- US-A- 4 052 176
- US-A- 4 070 165
- US-A- 4 449 994
- US-A- 4 784 672
- US-A- 5 451 249
- US-A1- 2007 028 772

## Description

### FIELD OF THE INVENTION

The present invention relates to the purification of biogas, such as that prepared by anaerobic digestion of waste material.

### BACKGROUND TO THE INVENTION

Biogas is produced by the anaerobic digestion or fermentation of wastewaters and solid residues. The material digested can include organic matter such as manure, sewage sludge, biodegradable waste or any other biodegradable feedstock.

The process is essentially the degradation of organic compounds in the absence of oxygen to produce a saturated gas mixture mainly of methane, carbon dioxide and hydrogen sulphide.

Before biogas can be used as fuel for vehicles it must be "upgraded" or enriched in methane to become bio-methane. Removal of most of the carbon dioxide raises the methane content sufficiently. Furthermore, the gas needs to be dried and hydrogen sulphide removed. In Sweden bio-methane has been used as a fuel since the early 1990s. The industry standard for bio-methane for vehicle fuel is the Swedish standard SS 15 54 38 - Standards A and B.

**Table 1: Excerpt from the Swedish Standard for biogas as vehicle fuel (SS 15 54 38)**

| **Component** | **Unit** | **Standard A** | **Standard B** |
|---|---|---|---|
| Methane, CH₄ | Vol-% | 96-98 | 95-99 |
| Water content | Mg/Nm³ | <32 | <32 |
| Oxygen, O₂ | Vol-% | <1 | <1 |
| Total sulphur | Mg/Nm³ | <23 | <23 |

We are particularly interested in the removal of hydrogen sulphide from biogas. Hydrogen sulphide is harmful to humans and animals. At lower concentrations the gas has an unpleasant odour. At higher concentrations it can present a threat to life.

Hydrogen sulphide in fuel gas is detrimental to metallic engine components and high pressure storage vessels due to its corrosive effect, especially in the presence of water. Hydrogen sulphide also has detrimental environmental effects including the formation of acid rain, when released via a vehicles exhaust.

Biogas upgrading is essentially any process which is capable of separating biogas into its constituent gases. Various techniques exist, the more relevant of which are outlined below.

"Water scrubbing" involves passing the biogas stream through an absorber or scrubber packed with media to provide a high contact surface area at a gas/ water interface.

The biogas is brought into contact with water at elevated pressure; components of the biogas are absorbed into the water until saturation equilibrium is reached. The gas which is not absorbed is discharged from the scrubber. This discharged gas is enriched in methane but also contains residual traces of hydrogen sulphide and carbon dioxide. This discharged gas is saturated with water.

Typical approximate contents are:
97% - 99% methane,
1%-2.5% carbon dioxide,
0.5% insoluble contaminants: (oxygen and nitrogen from the air dissolved in the scrubbing water or air contamination of the raw biogas stream),
5 PPM hydrogen sulphide,
Water vapour

The discharge gas remains unsuitable for immediate use due to its water saturation. Thus it is usually put through a drying process to remove water.

Draw backs to the water scrub process include clogging of packing due to the growth of bacteria, and the formation of elemental sulphur, especially when incoming biogas has a high level hydrogen sulphide in it.

"Polyethylene glycol scrubbing" is similar to water scrubbing. However there is a significantly higher propensity for the absorption of hydrogen sulphide and carbon dioxide. Therefore there is less demand for recirculation of the solvent, and the pumping costs are lower. However, as the polyethylene glycol scrubbing solution is regenerated by heating, energy costs may be higher than in other techniques.

"Pressure Swing Adsorption" (PSA) is an alternative technique used to separate carbon dioxide from biogas to increase the methane content. PSA separates particular species from a gas under pressure according to those species' molecular sizes. Porous materials are used as molecular sieves, such as zeolites. PSA works by adsorbing at one pressure, and then lowering the pressure to desorb gas from the porous materials.

Various problems exist with PSA including:
- Poisoning - where the porous media may become permanently saturated with a contaminant such as hydrogen sulphide, preventing its regeneration.
- Disposal of media - at the end of the useful life, or after poisoning the porous media needs to be replaced creating a disposal issue for the used media.
- Methane Loss - Methane gas losses to atmosphere are higher than those produced by the water scrub technique. This is considered a negative due to the higher propensity of methane to act as a greenhouse gas than carbon dioxide.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

### OBJECT OF THE INVENTION

It is an object of the present invention to provide an alternative or improved means of upgrading biogas to those currently available. It is an alternative object to overcome at least one of the abovementioned disadvantages.

### ACKNOWLEDGEMENT OF PRIOR ART

Examples of existing bio gas upgrading systems may be found in EP 0,180,670 A1, US 5,451,249 A, EP1,120,149 A1, and US 2007/028772 A1.

### SUMMARY OF THE INVENTION

According to a first aspect of the invention there is provided a process according to claim 1.

Preferably the wet scrubbing process is a water scrubbing process, preferably carried out in a water scrubber.

Preferably the upgraded biogas exiting the water scrubber is at a temperature between 2-10°C. Preferably the upgraded biogas entering the column is at a temperature between 2-10°C upon entry.

Preferably upon exiting the column the H₂S levels of the upgraded biogas are in parts per billion.

Preferably upon exiting the column the produced bio-methane meets the Swedish standard for biogas SS 15 54 38; preferably standard A, more preferably standard B.

Broadly according to a further aspect of the invention there is provided **apparatus according to claim 8.**

Preferably the wet scrubber is a water scrubber.

Preferably the apparatus includes two substantially identical columns arranged in parallel, allowing one column to be on line whilst the other column is regenerated.

Preferably the apparatus includes means to separate a fraction of the upgraded biogas exiting the on-line column, heating of that fraction of gas, and passage of that fraction of gas in a counter current direction through the column being regenerated.

Other aspects of the invention may become apparent from the following description which is given by way of example only and with reference to the accompanying drawings.

The term "comprising" as used in this specification means "consisting at least in part of". When interpreting statements in this specification which include that term, the features, prefaced by that term in each statement all need to be present, but other features can also be present. Related terms such as "comprise" and "comprised" are to be interpreted in the same manner.

The term "activated carbon" as used in this specification also includes the terms activated charcoal or activated coal.

The term "molecular sieves" refers to materials containing tiny pores of a precise and uniform size that is used as an absorbent for gases and liquids. These are often aluminosilicate materials but we are not restricted only to aluminosilicates.

The term "and/or" means either "and" or "or", or, where the context allows, both.

### BRIEF DESCRIPTION OF THE DRAWINGS

The invention will now be described by way of example only and with reference to the drawings in which:
**Figure 1****:** Schematic illustration of a column used to remove contaminants in accordance with the invention;
**Figure 2****:** Process flow diagram of one embodiment of the invention;

### DETAILED DESCRIPTION OF THE INVENTION

We have developed a process for the purification of biogas which reduces the levels of the impurities of a biogas feed. Raw biogas usually contains methane (CH₄) of around 65% carbon dioxide (CO₂) around 35% and other contaminants such as air, hydrogen sulphide (H₂S) and siloxanes.

Our invention is directed towards improved methane recovery and/or also to reducing the content of these other contaminants, and in preferred embodiments, particularly reducing the content of hydrogen sulphide.

### Biogas upgrading

Our invention uses a three step process to reduce the impurity content and in preferred embodiments particularly the H₂S content.
a) a first process of wet (preferably water) scrubbing to remove predominantly H₂S and CO₂;
b) a second process of dehydration using molecular sieves or other equivalent materials;
c) a third process of further H₂S removal using activated carbon.

The key features of our process which render it inventive include at least one of that:
- the process is a hybrid process of water scrubbing and PSA albeit in a non-obvious fashion, incorporating further steps;
- the second and third processes are, unusually, carried out in a single column or location;
- we get unusually high performance from our process in that levels of impurities (particularly H₂S) are significantly lower than what we have experienced previously.

### Water Scrubbing

The wet scrubbing process uses liquid to absorb the impurity gases from the biogas. We prefer to use water (i.e. water scrubbing) but alternatives to water can be used as is known in the art, such as amines or polyethylene glycol.

The scrubber operates at an elevated pressure to force gas absorption into water. The scrubber can operate at temperatures between just above 0°C - 30°C; however we prefer to operate between 2 - 10°C. The scrubbing process upgrades the biogas quality by preferentially absorbing the undesirable gases, such as CO₂ and H₂S into the water.

Our preferred arrangement for water scrubbing is described below.

### Dehydration

Following our water scrubbing process the gas is then subject to a dehydration process, using molecular sieve/porous materials as are known in the art. Type 4A and 13X molecular sieves are the common sieves used for water removal and are suitable here. They are able to remove water vapour and contaminants having a smaller critical diameter than 4A. However other suitable porous materials can be used as is well known by those skilled in the are.

Although drying of a gas stream using such materials is used in prior art biogas purification this has a number of unique features.

### Activated Carbon removal of H₂S

The upgraded biogas is then further purified by passing through activated carbon. It is not at elevated temperature but at the temperature of the gas (substantially same as 2-10°C). Activated carbon is known in the art to remove H₂S from gas systems. Any grade or brand or activated carbon capable of H₂S removal would be suitable.

### Dehydration and H₂S Removal in one location

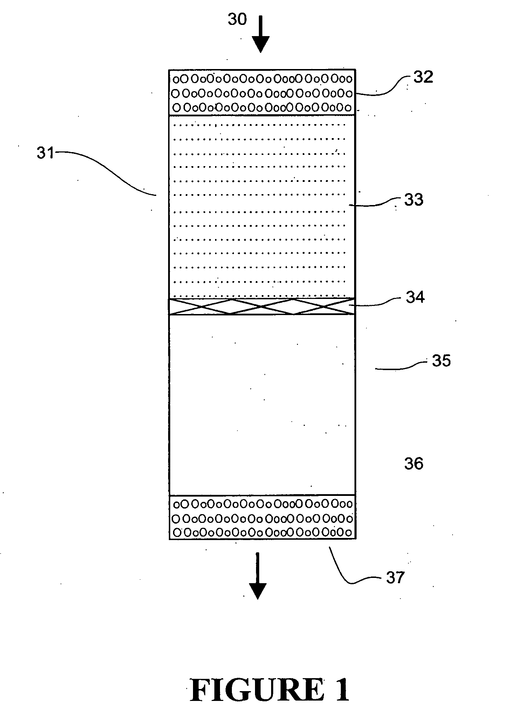

In our system we have devised a way that the dehydration column and activated charcoal bed can be housed in one location. The preferred form uses one column as illustrated in Figure 1. In the preferred form of the process the upgraded biogas 30 enters the column 31 at one end, passes through a layer of the pebbles 32 acting as a course mesh to keep the contents in place, and then to the dehydration stage via molecular sieves 33. The column is constructed with a dividing mesh of stainless steel 34 between the molecular sieves 33 (again to keep the contents in place) and the following activated carbon 35. The purified gas passes through a final layer of pebbles 36, and out the distal end 37 of the column 31.

### Advantages of the Invention

The invention possesses at least one of the following novel features and/or advantages.

Firstly the gas entering the dehydration process has been cooled by passing through the preceding wet scrubbing step. In this case the temperature of the incoming gas is approximately at the same temperature as the water scrubber operating temperature, likely between 2-10°C. This is cooler than the gases purified by PSA processes in the prior art. The gas temperature upon entry to a PSA column is generally around 30°C. One significant advantage of our operating lower temperature is that the water content of the incoming biogas is lower than that of gas at ambient temperature. This is an advantage for our method as the column can be on line for a longer period before needing regeneration. We believe the gas make up on entry to the column has 0.07%-0.12% water (vol), around 2% CO₂ and around 1-5ppm H₂S.

Secondly the proximity of the drying step to the following H₂S removal by activated carbon is unique.
i. we believe we are seeing a much greater degree of H₂S reduction than in the prior art we can deal with incoming gas streams having H₂S levels in the 500-2000ppm levels. Our levels of H₂S in our product gas are now parts per billion (ppb). After exiting the water scrubbing system, we see levels of less than 5ppm but it is the positioning and conditions or the subsequent purification column process which give the vast improvement to parts per billion observed.
ii. We have a greater efficiency of design by placing the dehydration column in the same place as the activated charcoal bed.
iii. Our net capture of methane is greater than that observed from processes of the prior art. We see losses of 1% product or less whilst conventional PSA process to 5% of their product due to their regeneration system.

### Regeneration of the Column

The molecular sieves and the activated carbon require regeneration. This is generally brought about by depressurisation and heating.

In practice a plant utilising our process may have two columns 31 allowing one column to be taken off line to regenerate while the other remains on-line.

The regenerating column is depressurised back to suction pressure (around atmospheric pressure), heated until regenerated, cooled, then re-pressurised. Depressurisation and heating increases the H₂S volatility.

In one embodiment a fraction of the product gas is taken, heated and sent back through the off line column to bring about the regeneration. It is important to note that in column regeneration we avoid any exposure of the column to oxygen as this could be a hazard.

In our system, as we have a comparatively lowly saturated gas entering the column in the first place (in comparison to standard PSA processes) we have a longer on line time of between 8-24 hours for each column. One advantage of our system is that we match residence times of the two media in each column (molecular sieves and activated carbon) - in other words, we used proportions of the two different media such that the possible saturation time of the purification beds through any column is substantially the same before regeneration is required. This is a considerable advantage over the prior art where one bed, either the molecular sieve or the activated carbon, may become saturated before the other is saturated, thus forcing a regeneration cycle prematurely.

When a new column comes on line, we obtain a product gas with a dew point around - 110°C at the start. This increases as the time the column is on line increases. Our switch point from one column to the other is at a dew point of -85°C. The dew point needs to be low as the biogas after processing is compressed to high pressure (250bar.g) as it is used as vehicle fuel. In freezing conditions the formation of water crystals, (ice) or methyl-hydrates is undesirable.

### EXAMPLES

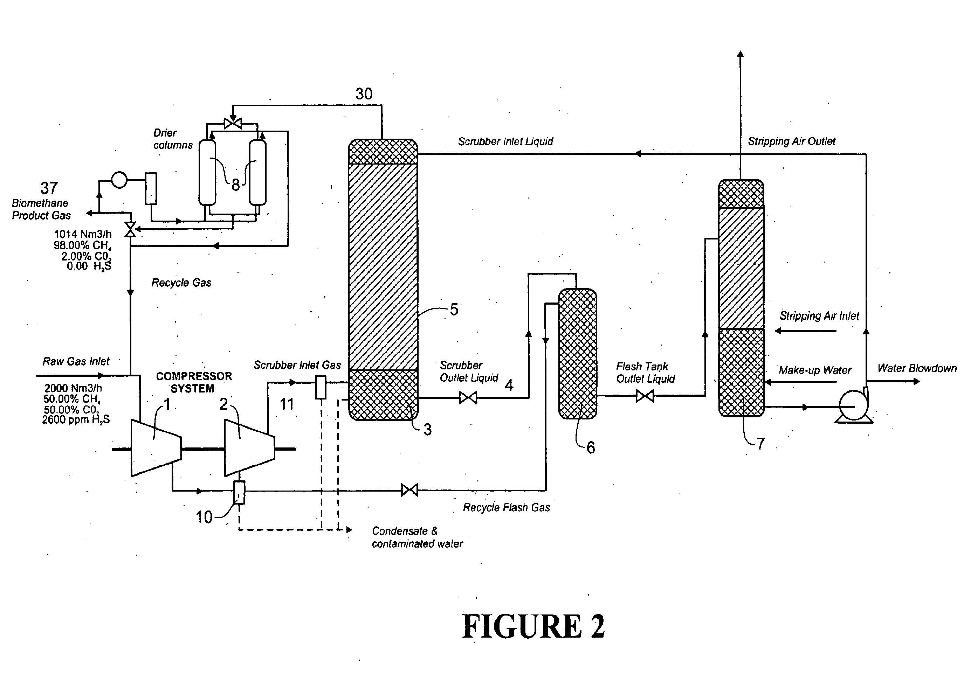

Figure 2 illustrates a process flow diagram of one preferred plant facility according to the invention. This facility is designed to upgrade raw biogas, through the removal of CO₂ and other soluble gases, to produce primarily methane gas that is ideally clean and dry, and suitable for use as vehicle fuel.

With reference to Figure 2 our overall system incorporates a gas compression system 1-2, a water scrubbing system 5, a flashing tank for gas recovery 6, a stripper vessel for regeneration of the process water 7 and a gas drying/purification system follows 8. Our preferred system is now discussed in detail.

### The biogas passes from the inlet separator to suction of a stage one compressor 1 and is compressed to approximately 2.5 bar(g).

A water-cooled shell and tube intercooler cools the biogas after stage one compression. A check valve is installed between the stage one compressor discharge and intercooler to prevent reverse flow of biogas when the system is stopped.

A condensate collector pot at the discharge of the intercooler collects and removes condensate and compressor lube oil from the biogas. This collector pot also acts as a receiver for the flash gas recovered from the flashing tank. **10**

The biogas passes from the intercooler to suction of the stage two compressor 2 and is compressed to approximately 9 bar(g).

Over pressure and over temperature protection devices are fitted on the stage two discharge.

A water-cooled shell and tube aftercooler cools the biogas after stage two compression. A check valve is installed between the stage two compressor discharge and aftercooler to prevent reverse flow of biogas when the system is stopped.

A condensate collector pot **11** at the discharge of the aftercooler collects and removes condensate and compressor lube oil from the biogas.

After the two stages of compression and cooling, the biogas enters the bottom of the scrubber vessel. **5** Our preferred scrubbing vessel incorporates a weir decant system (not shown) that skims off and removes the top layer of liquid in the scrubber 5 which may contain light hydrocarbon fractions, sulphur, fats and other contaminants. Inside the scrubber from 3, the biogas rises to the top, counter-flow to the process water flowing downwards. The water preferentially absorbs the undesirable gases such as CO₂ and H₂S, and product gas, which, is how mainly CH₄, exits from the top of the scrubber. **30**

Packing media and distributors inside the scrubber provide increased surface contact area between the gas and water, and maximise absorption efficiency.

The water discharged from the scrubber is saturated with dissolved CO₂, H₂S and a small amount of CH₄. It is necessary to regenerate the water by stripping it of these dissolved gases.

After being discharged from the scrubber, **4** the water is sent to the flashing tank 6. This tank 6 operates at an intermediate pressure. The pressure is lower than the scrubber pressure, but higher than the stripper pressure (and the stage one compressor discharge pressure). Inside the flashing tank 6, most of the CH₄ that was absorbed by the water in the scrubber is flashed off. This recovered flash gas is fed back into the stage one condensate collector pot **10.** A back pressure regulating valve on the flash gas line regulates the pressure in the flashing tank.

The water discharged from the flashing tank is delivered to the top of the stripper vessel **7.** After being cooled, the water is delivered to the top of the scrubber. A hold-up device and distributor at the top of the stripper ensures the water flows evenly down the stripper to maximise stripping efficiency.

Dissolved methane gas is removed in the flashing vessel 6. Water is regenerated by desorption of any remaining dissolved CO₂ gas in the stripping vessel 7. Water is re-pressurised and recirculated through the scrubber **5.**

After the scrubber, **5** the water saturated product gas passes to a coalescing separator vessel (not shown) to remove free moisture.

After the coalescing separator vessel, the product gas passes through a PSA/TSA (Pressure Swing/Temperature Swing Adsorption) drier **8.** Molecular sieve media in the drier columns adsorbs moisture and dries the product gas.

The drier is most preferably a vessel acting as a drying/purification system. Preferably this comprises of a Pressure Swing/Temperature Swing Adsorption (PSA/TSA) drier, which dries and further purifies the upgraded biogas after the scrubber, making it suitable for use as a vehicle fuel.

There most preferably the water saturated upgraded biogas from the scrubber 5 moves to a coalescing separator vessel (not shown) for removal of free moisture, then to the gas drying and H₂S removal system. A column of this system is as illustrated in Figure **2****.** This regenerative drying/purification system uses dual columns. One column is always active, drying and purifying the product gas. The other column is regenerating or on standby. Each column has the capacity to be actively drying and purifying for approximately 8 hours under full load before requiring regeneration.

In the preferred embodiment regeneration is brought about by depressurizing the column and then taking around 5% of the product methane, heating this gas stream and sending it back through the off-line column in a counter current direction.

The dried product gas passes through a filter and a back pressure control valve before being discharged. The filter traps any trace particles of molecular sieve media that may have been collected from the drier column. The control valve maintains a steady set pressure at the scrubber, thus ensuring consistent CO₂ and H₂S absorption.

The bio-methane product gas produced from the system consists of 97-98% CH₄, with H₂S of the order of parts per billion and dew point of less than -85°C.

### INDUSTRIAL APPLICATION

The method of the invention can be applied to biogas purification but also to other methane purification processes as would be envisaged by one skilled in the art.

Where in the foregoing description reference has been made to elements or integers having known equivalents, then such equivalents are included as if they were individually set forth.

Although the invention has been described by way of example and with reference to particular embodiments; it is to be understood that modifications and/or improvements may be made without departing from the scope of the invention as defined by the appended claims.

## Claims

1. A process for increasing the concentration of methane gas in a biogas stream **characterised in that** said process comprises the steps of
i) wet scrubbing the biogas stream (5),
ii) drying the thus upgraded biogas stream by passage through a porous material able to remove water from the stream (33) and comprising molecular sieves, and
iii) removing hydrogen sulphide, or at least some of the hydrogen sulphide, from the upgraded biogas stream by passage through activated carbon (35), **characterized in that**
the step of removal of the hydrogen sulphide is subsequent to the drying step ii); and
wherein the porous material and the activated carbon are located in a single vessel (31) and the drying and hydrogen sulphide removal steps are carried out by passage of the upgraded biogas through this vessel.

2. A process of claim 1 wherein the wet scrubbing step i) is a water scrubbing process in a water scrubber (5).

3. A process of claim 2 wherein the upgraded biogas stream exiting the water scrubber (5) is at a temperature between 2-10°C.

4. A process of claim 1 wherein the upgraded biogas entering the single vessel (31) is at a temperature between 2-10°C upon entry.

5. A process of claim 1 wherein upon exiting the single vessel (31) the hydrogen sulphide level of the upgraded biogas is below 5 parts per million.

6. A process of claim 1 wherein upon the gas stream exiting the single vessel comprises 95-99 Vol% Methane, less than 32 mg/Nm³ water, less than 1 Vol-% Oxygen and less than 23 mg/Nm³ Sulphur.

7. A process of claim 1 wherein steps ii) and iii) are performed in sequence in a bank of at least two such vessels (8) and the stream is diverted from one vessel to the other vessel as the content of a vessel degrades in use.

8. An apparatus for increasing the methane gas content and/or reducing the hydrogen sulphide of a wet stream of biogas **characterised in that** said apparatus comprises a wet scrubber (5) followed by an adsorber, the adsorber comprising a column **characterized in that** the column is packed sequentially with a gas dehydrating porous material (33) comprising molecular sieves followed by activated carbon (35).

9. Apparatus of claim 8 wherein the wet scrubber (5) contains water as a solvent.

10. Apparatus of claim 9 wherein the apparatus includes two identical columns arranged in parallel (8), allowing one column to be on line whilst the other column is regenerated.

11. Apparatus of claim 10 wherein there are means to separate a fraction of the upgraded biogas exiting the on-line column, heating of that fraction of gas, and means for passage of that fraction of gas in a counter current direction through the column being regenerated.

## Patentansprüche

1. Verfahren zum Erhöhen der Konzentration von Methangas in einem Biogasstrom, **dadurch gekennzeichnet, dass** das Verfahren die Schritte von
i) Nassreinigen des Biogasstroms (5),
ii) Trocknen des dadurch aufbereiteten Biogasstroms mittels Durchführen durch ein poröses Material, welches in der Lage ist, Wasser aus dem Strom zu entfernen (33) und Molekularsiebe umfasst, und
iii) Entfernen von Schwefelwasserstoff oder von zumindest einem Anteil des Schwefelwasserstoffs aus dem aufbereiteten Biogasstrom mittels Durchführens durch Aktivkohle (35),
**dadurch gekennzeichnet, dass**
der Schritt der Entfernung des Schwefelwasserstoffs auf den Trocknungsschritt ii) folgt; und
wobei das poröse Material und die Aktivkohle sich in einem einzelnen Gefäß (31) befinden und die Trocknungs- und Schwefelwasserstoffentfernungsschritte mittels Durchführens des aufbereiteten Biogases durch dieses Gefäß durchgeführt werden, umfasst.

2. Verfahren nach Anspruch 1, wobei der Nassreinigungsschritt i) ein Wasserreinigungsverfahren in einem Wasserreiniger (5) ist.

3. Verfahren nach Anspruch 2, wobei der aufbereitete Biogasstrom, der aus dem Wasserreiniger (5) austritt, bei einer Temperatur zwischen 2-10°C ist.

4. Verfahren nach Anspruch 1, wobei der aufbereitete Biogasstrom, der in das einzelne Gefäß (31) eintritt, beim Eintritt bei einer Temperatur zwischen 2-10°C ist.

5. Verfahren nach Anspruch 1, wobei der Schwefelwasserstoff-Level des aufbereiteten Biogases nach Austreten aus dem einzelnen Gefäß (31) unter fünf Teilen pro Million ist.

6. Verfahren nach Anspruch 1, wobei der Gasstrom nach Austreten aus dem einzelnen Gefäß 95-99 Vol.-% Methan, weniger als 32 mg/Nm³ Wasser, weniger als 1 Vol.-% Sauerstoff und weniger als 23 mg/Nm³ Schwefel umfasst.

7. Verfahren nach Anspruch 1, wobei Schritte ii) und iii) in Sequenz in einer Reihe von mindestens zwei solchen Gefäßen (8) durchgeführt werden und der Strom von einem Gefäß in das andere Gefäß umgeleitet wird, wenn der Inhalt eines Gefäßes bei Verwendung degradiert.

8. Vorrichtung zum Erhöhen des Methangasgehalts und/oder Reduzieren des Schwefelwasserstoffs eines nassen Biogasstroms, **dadurch gekennzeichnet, dass** die Vorrichtung einen Nassreiniger (5) gefolgt von einem Adsorber umfasst, wobei der Adsorber eine Säule umfasst, **dadurch gekennzeichnet, dass** die Säule sequenziell mit einem Gasdehydrierenden porösen Material (33), welches Molekularsiebe umfasst, gefolgt von Aktivkohle (35) gepackt ist.

9. Vorrichtung nach Anspruch 8, wobei der Nassreiniger (5) Wasser als Lösungsmittel enthält.

10. Vorrichtung nach Anspruch 9, wobei die Vorrichtung zwei identische Säulen, die parallel angeordnet sind (8), umfasst, was zulässt, dass eine Säule eingeschaltet ist, während die andere Säule regeneriert wird.

11. Vorrichtung nach Anspruch 10, wobei Mittel, um eine Fraktion des aufbereiteten Biogases, welches aus der eingeschalteten Säule austritt, abzutrennen, diese Gasfraktion zu erwärmen, und Mittel zum Durchführen dieser Gasfraktion in einer Gegenstromrichtung durch die Säule, welche regeneriert wird, vorhanden sind.

## Revendications

1. Procédé pour augmenter la concentration de gaz méthane dans un flux de biogaz, **caractérisé en ce que** ledit procédé comprend les étapes consistant à :
i) épurer par voie humide le flux de biogaz (5),
ii) sécher le flux de biogaz ainsi amélioré en le faisant passer à travers un matériau poreux pouvant retirer l'eau du flux (33) et comprenant des tamis moléculaires, et
iii) retirer le sulfure d'hydrogène ou au moins une partie du sulfure d'hydrogène, du flux de biogaz amélioré en le faisant passer à travers du charbon actif (35), **caractérisé en ce que** :
l'étape consistant à retirer le sulfure d'hydrogène a lieu après l'étape de séchage ii) ; et
dans lequel le matériau poreux et le charbon actif sont positionnés dans une cuve unique (31) et les étapes de séchage et de retrait du sulfure d'hydrogène sont réalisées en faisant passer le biogaz amélioré dans cette cuve.

2. Procédé selon la revendication 1, dans lequel l'étape d'épuration par voie humide i) est un procédé d'épuration à l'eau dans un épurateur à eau (5).

3. Procédé selon la revendication 2, dans lequel le flux de biogaz amélioré sortant de l'épurateur à eau (5) est à une température comprise entre 2 et 10°C.

4. Procédé selon la revendication 1, dans lequel le biogaz amélioré entrant dans la cuve unique (31) est à une température comprise entre 2 et 10°C, après l'entrée.

5. Procédé selon la revendication 1, dans lequel, après être sorti de la cuve unique (31), le niveau de sulfure d'hydrogène du biogaz amélioré est inférieur à 5 parts par million.

6. Procédé selon la revendication 1, dans lequel, après la sortie du flux de gaz, la cuve unique comprend 95-99 % en volume de méthane, moins de 32 mg/Nm³ d'eau, moins de 1 % en volume d'oxygène et moins de 23 mg/Nm³ de sulfure.

7. Procédé selon la revendication 1, dans lequel les étapes ii) et iii) sont réalisées en séquence dans une rangée d'au moins deux de ces cuves (8) et le flux est dévié d'une cuve à l'autre au fur et à mesure que le contenu d'une cuve se dégrade, à l'usage.

8. Appareil pour augmenter la teneur en gaz méthane et/ou réduire le sulfure d'hydrogène d'un flux humide de biogaz, **caractérisé en ce que** ledit appareil comprend un épurateur à voie humide (5) suivi d'un adsorbeur, l'adsorbeur comprenant une colonne, **caractérisé en ce que** la colonne est remplie séquentiellement avec un matériau poreux de déshydratation de gaz (33) comprenant des tamis moléculaires, suivis de charbon actif (35).

9. Appareil selon la revendication 8, dans lequel l'épurateur à voie humide (5) contient de l'eau en tant que solvant.

10. Appareil selon la revendication 9, dans lequel l'appareil comprend deux colonnes identiques agencées en parallèle (8), permettant à une colonne d'être alignée tandis que l'autre colonne est régénérée.

11. Appareil selon la revendication 10, dans lequel il y a des moyens pour séparer une fraction du biogaz amélioré sortant de la colonne alignée, chauffer cette fraction de gaz et des moyens pour faire passer cette fraction de gaz dans une direction à contre-courant à travers la colonne qui est régénérée.
